# DEMANDE DE BREVET EUROPEEN

(11) **EP 1 938 796 A1**
(43) Date de publication de la demande: **02.07.2008**
(21) Numéro de dépôt: 07123616.0
(22) Date de dépôt: 19.12.2007
(51) Int. Cl.: A61K 8/86, A61K 8/90, A61Q 3/02

(54) **Vernis à ongles comprenant un polycondensat et un copolymère polyoxyalkylène**

(30) Priorité: 22.12.2006 FR 0655881
(71) Demandeur: L'Oréal, 75008 Paris (FR)
(72) Inventeur: Guerchet, Laurence, 94550, CHEVILLY-LARUE (FR); Coffey-Dawe, Lizabeth-Anne, 93600, AULNAY SOUS BOIS (FR)
(74) Mandataire: Duvert, Sandra

(57) **Abrégé**

La présente invention a pour objet une composition de vernis à ongles comprenant, dans un milieu solvant organique :
a) au moins un polycondensat susceptible d'être obtenu par réaction :
- d'au moins un polyol comprenant 3 à 6 groupes hydroxyles;
- d'au moins un acide monocarboxylique non aromatique, saturé ou insaturé, linéaire, ramifié et/ou cyclique, comprenant 6 à 32 atomes de carbone;
- d'au moins un acide monocarboxylique aromatique comprenant 7 à 11 atomes de carbone,
- d'au moins un acide polycarboxylique, saturé ou insaturé, voire aromatique, linéaire, ramifié et/ou cyclique, comprenant au moins 2 groupes carboxyliques COOH et/ou un anhydride cyclique d'un tel acide polycarboxylique, et

b) au moins un copolymère bloc polyoxyalkyléne choisi parmi :
- les copolymères bloc de formule (I) suivante :

HO-(CH2-CH2-O)n-(C×H2×0)m-(CH2-CH2-O)p-H,

dans laquelle n,m,p sont indépendamment des nombres entiers allant de 1 à 1000 et x est un nombre entier strictement supérieur à 2 et inférieur ou égal à 10,
- les copolymères comprenant au moins un bloc polyéthylène glycol et au moins un bloc polypropylène glycol, ledit copolymère présentant un poids moléculaire supérieur ou égal à 1000 g/mol et leurs mélanges.

## Description

La présente invention a pour objet un vernis à ongles comprenant un copolymère bloc polyoxyalkylène et un polycondensat de type alkyde modifié. L'invention a également pour objet un procédé de maquillage ou de soin des ongles.

La composition de vernis à ongles peut être employée comme base pour vernis ou "base-coat" en terminologie anglo-saxonne, comme produit de maquillage des ongles, comme composition de finition, encore appelée "top-coat", à appliquer sur le produit de maquillage des ongles ou bien encore comme produit de soin cosmétique des ongles. Ces compositions peuvent s'appliquer sur les ongles d'êtres humains ou bien encore sur des faux ongles.

Les compositions de vernis à ongles comprennent en général des particules solides telles que les pigments, les nacres, les charges, qui sont en dispersion dans le milieu continu aqueux ou le milieu solvant organique de la composition.
Or ces particules ont tendance à sédimenter au cours du temps, du fait de leur densité qui est supérieure à celle du milieu continu dans lequel elles sont dispersées. Cette sédimentation se traduit par une modification de l'aspect macroscopique de la composition, et en particulier, dans le cas de vernis à ongles colorés, par une hétérogénéité de la couleur du vernis et une détérioration de la tenue du film de vernis sur les ongles.

Afin d'améliorer la stabilisation des compositions, le formulateur dispose d'agents épaississants ou gélifiants tels que par exemple la bentone ; toutefois, l'incorporation de ces agents en une quantité nécessaire à la stabilisation du vernis a tendance à matifier le film de vernis ce qui n'est pas souhaitable dans le cas des vernis à ongles où l'on cherche à obtenir un film brillant sur l'ongle.

Le demandeur a découvert de façon surprenante que l'association dans un vernis à ongles à milieu solvant organique, d'un polycondensat de type alkyde modifié et d'un copolymère bloc polyoxyalkylène particuliers permet l'obtention d'une composition de vernis à ongles qui présente une bonne stabilité et une bonne homogénéité de la couleur dans le temps, ainsi que la formation d'un film sur les ongles très brillant et présentant une bonne tenue dans le temps.

De façon plus précise, l'invention a pour objet une composition de vernis à ongles comprenant dans un milieu solvant organique :
a) au moins un polycondensat susceptible d'être obtenu par réaction :
   - de 15 à 30% en poids, par rapport au poids total du polycondensat, d'au moins un polyol comprenant 3 à 6 groupes hydroxyles;
   - de 5 à 40% en poids, par rapport au poids total du polycondensat, d'au moins un acide monocarboxylique non aromatique, saturé ou insaturé, linéaire, ramifié et/ou cyclique, comprenant 6 à 32 atomes de carbone;
   - de 10 à 55% en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
   - de 10 à 25% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique,
b) au moins un composé choisi parmi :
   - les copolymères bloc de formule (I) suivante :

      HO-(CH2-CH2-O)n-(CxH2xO)m-(CH2-CH2-O)p-H,

      dans laquelle n,m,p sont indépendamment des nombres entiers allant de 1 à 1000 et x est un nombre entier strictement supérieur à 2 et inférieur ou égal à 10,
   - les copolymères comprenant au moins un bloc polyéthylène glycol et au moins un bloc polypropylène glycol, ledit copolymère présentant un poids moléculaire supérieur ou égal à 1000 g/mol et leurs mélanges. L'association du copolymère bloc polyoxyalkylène et du polycondensat de type alkyde permet de formuler des compositions de vernis à ongles permettant l'obtention sur les ongles d'un film de brillance améliorée par rapport à l'état de l'art, tout en présentant une homogénéité satisfaisante et une bonne tenue.

La composition selon l'invention comprend un milieu cosmétiquement acceptable, c'est-à-dire un milieu non toxique et susceptible d'être appliqué sur la peau, les phanères ou les lèvres du visage d'êtres humains.

L'invention a aussi pour objet un procédé cosmétique de maquillage ou de soin non thérapeutique des ongles comprenant l'application sur les ongles d'au moins une couche d'une composition de vernis à ongles telle que définie ci-dessus.

L'invention a encore pour objet l'utilisation de ladite composition pour obtenir, après application sur les ongles, un film de vernis brillant..

### 1/ Polycondensat

Le polycondensat présent dans la composition selon l'invention est susceptible d'être obtenu par réaction :
- de 15 à 30% en poids, par rapport au poids total du polycondensat, d'au moins un polyol comprenant 3 à 6 groupes hydroxyles;
- de 5 à 40% en poids, par rapport au poids total du polycondensat, d'au moins un acide monocarboxylique non aromatique saturé, linéaire, ramifié et/ou cyclique, comprenant 6 à 32 atomes de carbone;
- de 10 à 55% en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 10 à 25% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique. Les polycondensats de la composition selon l'invention sont avantageusement branchés (ramifiés); on peut penser que ceci permet de générer un réseau par enchevêtrement des chaînes polymériques, et donc d'obtenir les propriétés recherchées, notamment en terme de tenue améliorée et en terme de solubilité. On a en effet constaté que les polycondensats linéaires ne permettaient pas d'obtenir une amélioration notable de la tenue de la composition, et que les polycondensats de type dendrimères, dont les chaînes sont régulières, ne présentaient pas une solubilité optimale.

Le polycondensat peut être présent dans la composition selon l'invention en une teneur en matières sèches allant de 0,5 à 50% en poids, de préférence allant de 0,5 à 40% en poids, notamment de 1 à 25% en poids, et mieux de 5 à 20% en poids, par rapport au poids de la composition finale.

Les polycondensats selon l'invention sont des polycondensats de type alkyde, et sont donc susceptibles d'être obtenus par estérification/polycondensation, selon les méthodes connues de l'homme du métier, des constituants décrits ci-après.

L'un des constituants nécessaires pour la préparation des polycondensats selon l'invention est un composé comprenant 3 à 6 groupes hydroxyles (polyol), notamment 3 à 4 groupes hydroxyles. On peut bien évidemment utiliser un mélange de tels polyols.
Ledit polyol peut notamment être un composé carboné, notamment hydrocarboné, linéaire, ramifié et/ou cyclique, saturé ou insaturé, comprenant 3 à 18 atomes de carbone, notamment 3 à 12, voire 4 à 10 atomes de carbone, et 3 à 6 groupes hydroxy (OH), et pouvant comprendre en outre un ou plusieurs atomes d'oxygène intercalés dans la chaîne (fonction éther).
Ledit polyol est de préférence un composé hydrocarboné saturé, linéaire ou ramifié, comprenant 3 à 18 atomes de carbone, notamment 3 à 12, voire 4 à 10 atomes de carbone, et 3 à 6 groupes hydroxy (OH).

Il peut être choisi parmi, seul ou en mélange :
- les triols, tels que le 1,2,6-hexanetriol, le triméthyloléthane, le triméthylolpropane, le glycérol;
- les tétraols, tels que le pentaérythritol (tétraméthylolméthane), l'érythritol, le diglycérol ou le ditriméthylolpropane;
- les pentols tels que le xylitol,
- les hexols tels que le sorbitol et le mannitol; ou encore le dipentaérythritol ou le triglycérol.
   De préférence, le polyol est choisi parmi le glycérol, le pentaérythritol, le sorbitol et leurs mélanges; et encore mieux est du pentaérythritol.

Le polyol, ou le mélange de polyol, représente de préférence 15 à 30% en poids, notamment 16 à 28% en poids, et mieux 18 à 25% en poids, du poids total du polycondensat final.

Un autre constituant nécessaire pour la préparation des polycondensats selon l'invention est un acide monocarboxylique non aromatique, saturé ou insaturé, linéaire, ramifié et/ou cyclique, comprenant 6 à 32 atomes de carbone, notamment 8 à 28 atomes de carbone et encore mieux 10 à 20, voire 12 à 18, atomes de carbone. On peut bien évidemment utiliser un mélange de tels acides monocarboxyliques non aromatiques.
Par acide monocarboxylique non aromatique, on entend un composé de formule RCOOH, dans laquelle R est un radical hydrocarboné saturé ou insaturé, linéaire, ramifié et/ou cyclique, comprenant 5 à 31 atomes de carbone, notamment 7 à 27 atomes de carbone, et encore mieux 9 à 19 atomes de carbone, voire 11 à 17 atomes de carbone.
De préférence, le radical R est saturé. Encore mieux, ledit radical R est linéaire ou ramifié, et préférentiellement en C5-C31.

Parmi les acides monocarboxyliques non aromatiques susceptibles d'être employés, on peut citer, seul ou en mélange, :
- les acides monocarboxyliques saturés tels que l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
- les acides monocarboxyliques insaturés mais non aromatiques, tels que l'acide caproléique, l'acide undécylénique, l'acide dodécylénique, l'acide myristoléique, l'acide palmitoléique, l'acide oléique, l'acide élaidique, l'acide gondoïque, l'acide érucique.
De préférence, on peut utiliser l'acide 2-éthylhexanoïque, l'acide isooctanoïque, l'acide laurique, l'acide palmitique, l'acide isostéarique, et leurs mélanges, et encore mieux l'acide isostéarique seul.

Ledit acide monocarboxylique non aromatique, ou le mélange desdits acides, représente de préférence 5 à 40% en poids, notamment 8 à 38% en poids, et mieux 10 à 35% en poids, du poids total du polycondensat final.

Un autre constituant nécessaire pour la préparation des polycondensats selon l'invention est un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone, notamment 2 à 12, voire 3 à 8 atomes de carbone.
On peut bien évidemment utiliser un mélange de tels acides monocarboxyliques aromatiques.
Par acide monocarboxylique aromatique, on entend un composé de formule R'COOH, dans laquelle R' est un radical hydrocarboné aromatique, comprenant 6 à 10 atomes de carbone, et en particulier les radicaux benzoïque et naphtoïque.
Ledit radical R' peut en outre être substitué par 1 à 3 radicaux alkyles, saturés ou insaturés, linéaires, ramifiés et/ou cycliques, comprenant 1 à 32 atomes de carbone, notamment 2 à 12, voire 3 à 8 atomes de carbone; et notamment choisis parmi méthyle, éthyle, propyle, isopropyle, butyle, isobutyle, terbutyle, pentyle, isopentyle, néopentyle, cyclopentyle, hexyle, cyclohexyle, heptyle, isoheptyle, octyle ou isooctyle.

Parmi les acides monocarboxyliques aromatiques susceptibles d'être employés, on peut citer, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque.
De préférence, on peut utiliser l'acide benzoïque, l'acide o-toluique, l'acide m-toluique, l'acide 1-naphtoïque, seuls ou en mélanges; et encore mieux l'acide benzoïque seul.

Ledit acide monocarboxylique aromatique, ou le mélange desdits acides, représente de préférence 10 à 55% en poids, notamment 20 à 52% en poids, voire 22 à 52% en poids, et mieux 25 à 50% en poids, du poids total du polycondensat final.

Un autre constituant nécessaire pour la préparation des polycondensats selon l'invention est un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique. On peut bien évidemment utiliser un mélange de tels acides polycarboxyliques et/ou d'anhydrides.

Ledit acide polycarboxylique peut notamment être choisi parmi les acides polycarboxyliques linéaires, ramifiés et/ou cycliques, saturés ou insaturés, voire aromatiques, comprenant 2 à 20 atomes de carbone, notamment 3 à 18, et encore mieux 4 à 12 atomes de carbone, voire 4 à 10 atomes de carbone; ledit acide comprend au moins deux groupes carboxyliques COOH, de préférence de 2 à 4 groupes COOH.
De préférence, ledit acide polycarboxylique est aliphatique saturé, linéaire et comprend 2 à 20 atomes de carbone, notamment 3 à 18 atomes de carbone, voire 4 à 12 atomes de carbone; ou bien est aromatique et comprend 8 à 12 atomes de carbone. Il comprend de préférence 2 à 4 groupes COOH.

Ledit anhydride cyclique d'un tel acide polycarboxylique peut notamment répondre à l'une des formules suivantes : 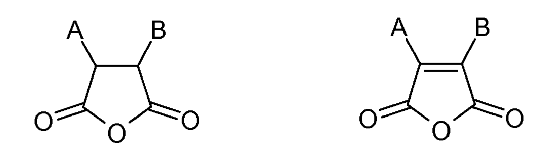 dans lesquelles les groupements A et B sont, indépendamment l'un de l'autre, :
- un atome d'hydrogène,
- un radical carboné, aliphatique, saturé ou insaturé, linéaire, ramifié et/ou cyclique, ou bien aromatique; comprenant 1 à 16 atomes de carbone, notamment 2 à 10 atomes de carbone, voire 4 à 8 atomes de carbone, notamment méthyle ou éthyle;
- ou bien A et B pris ensemble forment un cycle comprenant au total 5 à 7, notamment 6 atomes de carbone, saturé ou insaturé, voire aromatique.
De préférence, A et B représentent un atome d'hydrogène ou forment ensemble un cycle aromatique comprenant au total 6 atomes de carbone.

Parmi les acides polycarboxyliques ou leurs anhydrides, susceptibles d'être employés, on peut citer, seul ou en mélange :
- les acides dicarboxyliques tels que l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque;
- les acides tricarboxyliques tels que l'acide cyclohexanetricarboxylique, l'acide trimellitique, l'acide 1,2,3-benzènetricarboxylique, l'acide 1,3,5-benzènetricarboxy-lique;
- les acides tétracarboxyliques tels que l'acide butanetétracarboxylique et l'acide pyroméllitique,
- les anhydrides cycliques de ces acides et notamment l'anhydride phtalique, l'anhydride trimellitique, l'anhydride maléïque et l'anhydride succinique.
De préférence, on peut utiliser l'anhydride phtalique et/ou l'acide isophtalique, et encore mieux l'acide isophtalique seul.

Ledit acide polycarboxylique et/ou son anhydride cyclique, représente de préférence 10 à 25% en poids, notamment 11 à 22% en poids, et mieux 12 à 20% en poids, du poids total du polycondensat final.

Le polycondensat selon l'invention peut en outre comprendre une silicone à fonction hydroxyle (OH) et/ou carboxylique (COOH).
Elle peut comprendre 1 à 3 fonctions hydroxyle et/ou carboxylique, et comprend de préférence deux fonctions hydroxyle ou bien deux fonctions carboxyliques.
Ces fonctions peuvent être situées en bout de chaîne ou dans la chaîne, mais avantageusement en bout de chaîne.
On emploie de préférence des silicones ayant une masse moléculaire moyenne en poids (Mw) comprise entre 300 et 20000, notamment 400 et 10 000, voire 800 et 4000.

Cette silicone peut être de formule : 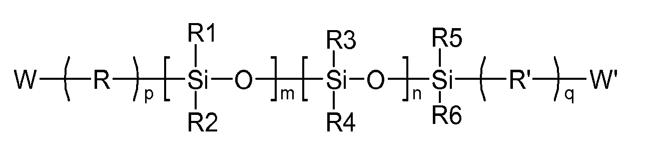 dans laquelle :
- W et W' sont, indépendamment l'un de l'autre, OH ou COOH; de préférence W=W';
- p et q sont, indépendamment l'un de l'autre, égaux à 0 ou 1,
- R et R' sont, indépendamment l'un de l'autre, un radical divalent carboné, notamment hydrocarboné, saturé ou insaturé, voire aromatique, linéaire, ramifié et/ou cyclique; comprenant 1 à 12 atomes de carbone, notamment 2 à 8 atomes de carbone, et comprenant éventuellement en outre 1 ou plusieurs hétéroatomes choisis parmi O, S et N, notamment O (éther);
   notamment R et/ou R' peuvent être de formule -(CH₂)ₐ- avec a=1-12, et notamment méthylène, éthylène, propylène, phénylène;
   ou bien de formule -[(CH₂)ₓO]_{z}- avec x = 1, 2 ou 3 et z = 1-10; en particulier x=2 ou 3 et z=1-4; et mieux x=3 et z=1.
- R1 à R6 sont, indépendamment l'un de l'autre, un radical carboné linéaire, ramifié et/ou cyclique, saturé ou insaturé voire aromatique; comprenant 1 à 20 atomes de carbone, notamment 2 à 12 atomes de carbone; de préférence, R1 à R6 sont saturés ou bien aromatiques, et peuvent notamment être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
- m et n sont, indépendamment l'un de l'autre, des entiers compris entre 1 et 140, et sont tels que la masse moléculaire moyenne en poids (Mw) de la silicone est comprise entre 300 et 20 000, notamment entre 400 et 10 000, voire entre 800 et 4000.

On peut notamment citer les polyalkylsiloxanes α,ω-diol ou α,ω-dicarboxylique, et notamment les polydiméthysiloxanes α,ω-diol et les polydiméthylsiloxanes α,ω-dicarboxylique; les polyarylsiloxanes α,ω-diol ou α,ω-dicarboxylique et notamment les polyphénylsiloxanes α,ω-diol ou α,ω-dicarboxylique; les polyarylsiloxanes à fonctions silanol tels que le polyphénylsiloxane; les polyalkylsiloxanes à fonctions silanol tels que le polydiméthylsiloxane; les polyaryl/alkylsiloxanes à fonctions silanol tels que le polyphényl/méthylsiloxane ou encore le polyphényl/propylsiloxane.
Tout particulièrement, on utilisera les polydiméthysiloxanes α,ω-diol de masse moléculaire moyenne en poids (Mw) comprise entre 400 et 10 000, voire entre 500 et 5000, et notamment entre 800 et 4000.

Lorsqu'elle est présente, ladite silicone peut de préférence représenter 0,1 à 15% en poids, notamment 1 à 10% en poids, voire 2 à 8% en poids, du poids du polycondensat.

Dans un mode de réalisation préféré de l'invention, l'acide monocarboxylique aromatique est présent en quantité molaire supérieure ou égale à celle de l'acide monocarboxylique non aromatique; notamment le rapport entre le nombre de mole d'acide monocarboxylique aromatique et le nombre de mole d'acide monocarboxylique non aromatique est de préférence compris entre 1,2 et 8, en particulier entre 1,3 et 7,8, voire entre 1,4 et 7,5 et encore mieux entre 1,9 et 7.
On a constaté que cela permet notamment d'obtenir un polymère avantageusement soluble dans les esters dits courts (type acétate de butyle ou d'éthyle) généralement employés pour formuler des compositions cosmétiques de type vernis à ongles; par ailleurs, le film obtenu présente une rigidité adéquate pour son utilisation dans les formules de vernis à ongles.

De préférence, le polycondensat selon l'invention est susceptible d'être obtenu par réaction :
- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol;
   présent de préférence en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
   présent de préférence en une quantité de 5 à 40% en poids, notamment 8 à 38% en poids, et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque;
   présent de préférence en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque et l'anhydride succinique;
   présent de préférence en une quantité de 10 à 25% en poids, notamment 11 à 22% en poids, et mieux 12 à 20% en poids, par rapport au poids total du polycondensat final.

Préférentiellement, le polycondensat selon l'invention est susceptible d'être obtenu par réaction :
- d'au moins un polyol choisi parmi, seul ou en mélange, le glycérol, le pentaérythritol, le sorbitol et leurs mélanges, et encore mieux le pentaérythritol seul; présent en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide 2-éthylhexanoïque, l'acide isooctanoïque, l'acide laurique, l'acide palmitique, l'acide isostéarique, et leurs mélanges, et encore mieux l'acide isostéarique seul; présent en une quantité de 5 à 40% en poids, notamment 8 à 38% en poids, et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide 1-naphtoïque, et encore mieux l'acide benzoïque seul; présent en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul ou en mélange, l'anhydride phtalique et l'acide isophtalique, et encore mieux l'acide isophtalique seul; présent en une quantité de 10 à 25% en poids, notamment 11 à 22% en poids, et mieux 12 à 20% en poids, par rapport au poids total du polycondensat final.

De préférence, le polycondensat selon l'invention présente :
- un indice d'acide, exprimé en mg d'hydroxyde de potassium par g de polycondensat, supérieur ou égal à 8; notamment compris entre 8 et 40, et encore mieux compris entre 10 et 30; et/ou
- un indice d'hydroxyle exprimé en mg d'hydroxyde de potassium par g de polycondensat, supérieur ou égal à 30; notamment compris entre 30 et 100, et encore mieux compris entre 40 et 90.
   Ces indices d'acide et d'hydroxyle peuvent être aisément déterminés par l'homme du métier par les méthodes analytiques habituelles.

De préférence, le polycondensat selon l'invention présente une viscosité, mesurée à 110°C, comprise entre 75 et 6000 mPa.s, notamment entre 80 et 5500 mPa.s, voire entre 90 et 5000 mPa.s, et encore mieux entre 200 et 4800 mPa.s. Cette viscosité est mesurée de la manière décrite avant les exemples.

Par ailleurs, le polycondensat est avantageusement soluble dans les esters courts, comprenant au total 3 à 8 atomes de carbone, notamment les acétates d'acides carboxyliques en C1-C6, et en particulier l'acétate de butyle et/ou l'acétate d'éthyle.
Par soluble, on entend que le polymère forme une solution limpide dans l'acétate de butyle ou l'acétate d'éthyle, à raison d'au moins 50% en poids, à 25°C; de préférence, le polymère selon l'invention est soluble à raison d'au moins 70% en poids dans l'acétate de butyle ou l'acétate d'éthyle.

De préférence, la solution du polymère selon l'invention dans l'acétate de butyle ou l'acétate d'éthyle, à 25°C, à une concentration de 70% en poids, présente une viscosité comprise entre 100 et 1500 mPa.s, notamment entre 120 et 900 mPa.s. La méthode de mesure est donnée avant les exemples.

Le polycondensat selon l'invention peut être préparé par les procédés d'estérification/polycondensation usuellement employés par l'homme du métier. A titre d'illustration, un procédé général de préparation consiste :
- à mélanger le polyol et les acides monocarboxyliques aromatiques et non aromatiques,
- à chauffer le mélange sous atmosphère inerte, d'abord jusqu'à la température de fusion (généralement 100-130°C) et ensuite à une température comprise entre 150 et 220°C jusqu'à consommation complète des acides monocarboxyliques (atteint lorsque l'indice d'acide est inférieur ou égal à 1), de préférence en distillant au fur et à mesure l'eau formée, puis
- à éventuellement refroidir le mélange à une température comprise entre 90 et 150°C,
- à ajouter l'acide polycarboxylique et/ou l'anhydride cyclique, et optionnellement la silicone à fonctions hydroxyles ou carboxyliques, en une seule fois ou de façon séquencée, puis
- à chauffer à nouveau à une température inférieure ou égale à 220°C, notamment comprise entre 170 et 220°C, de préférence en continuant à éliminer l'eau formée, jusqu'à l'obtention des caractéristiques requises en terme d'indice d'acide, de viscosité, d'indice d'hydroxyle et de solubilité.

Il est possible d'ajouter des catalyseurs d'estérification conventionnels, par exemple de type acide sulfonique (notamment à une concentration pondérale comprise entre 1 et 10%) ou type titanate (notamment à une concentration pondérale comprise entre 5 et 100 ppm).
Il est également possible de réaliser la réaction, en tout ou en partie, dans un solvant inerte tel que le xylène et/ou sous une pression réduite, pour faciliter l'élimination de l'eau.
Avantageusement, on n'utilise ni catalyseur ni solvant.

Ledit procédé de préparation peut comprendre en outre une étape d'addition d'au moins un agent antioxydant dans le milieu réactionnel, notamment à une concentration pondérale comprise entre 0,01 et 1%, par rapport au poids total de monomères, de façon à limiter les éventuelles dégradations liées à un chauffage prolongé.
L'agent antioxydant peut être de type primaire ou de type secondaire, et peut être choisi parmi les phénols encombrés, les amines secondaires aromatiques, les composés organophosphorés, les composés soufrés, les lactones, les bisphénols acrylés; et leurs mélanges.
Parmi les antioxydants particulièrement préférés, on peut notamment citer le BHT, le BHA , le TBHQ, le 1,3,5-trimethyl-2,4,6,tris(3,5-di-tertbutyl-4-hydroxybenzyl)-benzène, l'octadecyl-3,5,di-tertbutyl-4-hydroxycinnamate, le tetrakis-methylene-3-(3,5-di-tertbutyl-4-hydroxyphenyl)propionate méthane, l'octadecyl-3-(3,5-di-tertbutyl-4-hydroxyphenyl)propionate 2,5-di-tertbutyl hydroquinone, le 2,2-methyl-bis-(4-methyl-6-tertbutyl phénol), le 2,2-methylene-bis-(4-ethyl-6-tertbutyl phénol), le 4,4-butylidene-bis(6-tertbutyl-m-cresol), le N, N-hexamethylene bis(3,5-di-tertbutyl-4-hydroxyhydrocinnamamide), le pentaerythritol tetrakis (3-(3,5-di-tertbutyl-4-hydroxyphenyl)propionate) notamment celui commercialisé par CIBA sous le nom IRGANOX 1010; l'octadecyl 3-(3,5-di-tertbutyl-4-hydroxphenyl) propionate notamment celui commercialisé par CIBA sous le nom IRGANOX 1076; la 1,3,5-tris(3,5-di-tertbutyl-4-hydroxybenzyl)-1,3,5-triazine-2,4,6(1H,3H,5H)trione notamment celui commercialisée par Mayzo of Norcross, Ga sous le nom BNX 3114; le di(stearyl)pentaerythritol diphosphite, le tris(2,4-ditertbutyl phenyl)phosphite notamment celui commercialisé par CIBA sous le nom IRGAFOS 168; le dilauryl thiodipropionate notamment celui commercialisé par CIBA sous le nom IRGANOX PS800; le bis(2,4-di-tertbutyl)pentaerythritol diphosphite notamment celui commercialisé par CIBA sous le nom IRGAFOS 126; le bis(2,4-bis)[2-phénylpropan-2-yl]phényl)pentaérythritol diphosphite, le triphénylphosphite, le (2,4-di-tertbutylphenyl)pentaerythritol diphosphite notamment celui commercialisé par GE Specialty Chemicals sous le nom ULTRANOX 626; le tris(nonylphenyl)phosphite notamment celui commercialisé par CIBA sous le nom IRGAFOS TNPP; le mélange 1:1 de N,N-hexamethylenebis(3,5-di-tertbutyl-4-hydroxy-hydrocinnamamide) et de tris(2,4-di-tertbutylphenyl)phosphate notamment celui commercialisé par CIBA sous le nom Irganox B 1171; le tétrakis (2,4-di-tert-butylphényl)phosphite notamment celui commercialisé par CIBA sous le nom IRGAFOS P-EPQ; le distéarylthiodipropionate notamment celui commercialisé par CIBA sous le nom IRGANOX PS802; le 2,4-bis(octylthiométhyl)o-crésol notamment celui commercialisé par CIBA sous le nom IRGANOX 1520; le 4,6-bis(dodécylthiométhyl)o-crésol notamment celui commercialisé par CI BA sous le nom IRGANOX 1726.

### 2/ Copolymère bloc polyoxyalkylène

Par copolymère bloc, on entend un polymère comprenant au moins 2 blocs ou séquences distinctes, de préférence au moins 3 séquences distinctes.

Le copolymère bloc de la composition selon l'invention peut être choisi parmi les composés de formule (I) suivante :

HO-(CH2-CH2-O)n-(CxH2xO)m-(CH2-CH2-O)p-H,

dans laquelle n,m,p sont indépendamment des nombres entiers allant de 1 à 1000 et x est un nombre entier strictement supérieur à 2 et inférieur ou égal à 10.

Selon un mode de réalisation, le copolymère bloc comprend au moins un bloc polyéthylène glycol et au moins un bloc polypropylène glycol. Il peut alors répondre à la formule suivante :

HO-(CH2-CH2-O)n-(CxH2xO)m-H

avec n et m des nombres entiers entre 1 et 1000 et x un nombre entier supérieur strictement à 2 et inférieur à 10.

Le copolymère bloc de la composition présente avantageusement un poids moléculaire supérieur ou égal à 1000 g/mol, de préférence supérieur ou égal à 1500 g/mol, mieux supérieur à 2000 g/mol.

A titre de copolymère utilisables dans la composition selon l'invention, on peut citer les copolymères d'oxyde de propylène et d'oxyde d'éthylène, également appelés polycondensats OE/OP comme par exemple les polycondensats tribloc polyéthylène glycol / polypropylène glycol / polyéthylène glycol vendus sous les dénominations "SYNPERONIC" comme les "SYNPERONIC PE/ L44", le SYNPERONIC PE/ L64 (13 OE/13OP/130E) de PM 2900 et "SYNPERONIC PE/F127 " par la société ICI, et leurs mélanges.

Le ou les copolymères blocs polyoxyalkylène peu(ven)t être présent(s) en une quantité en matières sèches allant de 0,1 à 15% en poids par rapport au poids total de la composition, de préférence de 0,5 à 10%, et mieux de 1 à 8% en poids.

### Milieu solvant organique

La composition selon l'invention comprend un milieu solvant organique comprenant un solvant organique ou un mélange de solvants organiques.

Le solvant organique peut être choisi parmi :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane;
- les alkyl sulfoxides tels que le diméthylsulfoxide ;
- les aldéhydes liquides à température ambiante tels que le benzaldéhyde, l'acétaldéhyde;
- le 3-éthoxypropionate d'éthyle ;
- les carbonates tel que le carbonate de propylène, le carbonate de diméthyle ,
- les acétals tels que méthylal ;
- et leurs mélanges.

De préférence, le solvant est choisi parmi les esters à chaîne courte ayant de 3 à 8 atomes de carbone au total, tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle et leurs mélanges.

Le milieu solvant organique peut représenter de 10 à 95% en poids, par rapport au poids total de la composition, de préférence de 15% à 80% en poids, et mieux de 60 à 80% en poids.

La composition selon l'invention peut éventuellement comprendre un milieu aqueux, dans ce cas, le milieu aqueux est présent en une teneur inférieure ou égale à 2% en poids par rapport au poids total de la composition, de préférence inférieure ou égale à 1 % en poids.

### Polymère filmogène

La composition peut comprendre avantageusement un polymère filmogène, différent du polycondensat de type alkyde décrit plus haut.
Selon la présente invention, on entend par "polymère filmogène", un polymère apte à former à lui seul ou en présence d'un agent auxiliaire de filmification, un film continu et adhérent sur un support, notamment sur les matières kératiniques.
Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines polyester issues par exemple de la polycondensation d'acide adipique, d'un polyalcool comprenant de préférence de 2 à 6 atomes de carbone et d'un composé comportant trois fonctions acide et/ou anhydride et possédant de préférence de 8 à 16 atomes de carbone, les résines issues des produits de condensation d'aldéhyde tels que les résines cétone/formaldéhyde hydrogénée, les résines arylsulfonamide formaldéhyde comme la résine toluène sulfonamide formaldéhyde, les résines aryl-sulfonamide époxy ou encore les résines éthyl tosylamide.

Comme polymère filmogène, on peut notamment utiliser la nitrocellulose RS 1/8 sec ; RS ¼ sec. ; ½ sec. ; RS 5 sec. ; RS 15 sec. ; RS 35 sec. ; RS 75 sec.; RS 150 sec ; AS ¼ sec. ; AS ½ sec. ; SS ¼ sec. ; SS ½ sec. ; SS 5 sec., notamment commercialisée par la société HERCULES ; les résines polyesters de nom CTFA "copolymère d'acide adipique/néopentylglycol/anhydride trimellitique" comme l'Uniplex 670-P de UNITEX CHEMICAL CORPORATION, une résine cétone/formaldéhyde hydrogénée telle que celle commercialisée sous la dénomination Synthetic resin SK par la société DEGUSSA, les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Avantageusement, les première et deuxième séquences et du polymère séquencé sont incompatibles l'une avec l'autre.
De tels polymères sont décrits par exemple dans les documents EP 1411069 ou WO04/028488.

Le(s) polymère(s) filmogène(s) peu(ven)t être présent(s) dans la composition selon l'invention en une teneur totale en matières sèches allant de 0,1 % à 60 % en poids, par rapport au poids total de la composition, de préférence allant de 2 % à 50 % en poids, et mieux de 5 % à 30 % en poids.

### Agent auxiliaire de filmification

Pour améliorer les propriétés filmogènes de la composition de vernis à ongles un agent auxiliaire de filmification peut être prévu.
Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).
Ainsi, la composition peut comprendre, en outre, au moins un agent plastifiant et/ou un agent de coalescence. En particulier, on peut citer, seuls ou en mélange, les plastifiants et agents de coalescence usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les polyéthylène glycols, les polypropylène glycols, les copolymères polyéthylène glycols-polypropylène glycols différents du copolymère polyoxyalkylène décrit plus haut et leurs mélanges, notamment les polypropylène glycols de haut poids moléculaire, ayant par exemple une masse moléculaire allant de 500 à 15000, tels que par exemple
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther;
- des esters d'acides, notamment carboxyliques, tels que des citrates, des phtalates, des adipates, des carbonates, de tartrates, des phosphates, des sébaçates ;
- les esters issus de la réaction d'un acide monocarboxylique de formule R₁₁COOH avec un diol de formule HOR₁₂OH avec R₁₁ et R₁₂, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O, S, en particulier le monoester résultant de la réaction de l'acide isobutyrique et d'octanediol tel que le triméthyl-2,2,4 pentane diol 1,3, tel que celui commercialisé sous la référence TEXANOL Ester Alcohol par la société Eastman Chemical,
- des dérivés oxyéthylénés, tels que les huiles oxyéthylénées, notamment les huiles végétales, telles que l'huile de ricin ;
- les dimethicone copolyols, notamment à groupements α-ω propyl polyoxypropylène et
- leurs mélanges.

L'agent auxiliaire de filmification peut représenter de 0,01 à 40%, de préférence de 0,05 à 30% et mieux de 0,1 à 20% en poids par rapport au poids total de la composition.

### Agent épaississant

La composition peut comprendre, outre le copolymère bloc, un agent épaississant qui peut en particulier être choisi parmi : les silices notamment hydrophobes, telles que celles décrites dans le document EP-A-898960, et par exemple commercialisées sous les références "AEROSIL R812 par la société Degussa, "CAB-O-SIL TS-530^{®}", "CAB-O-SIL TS-610^{®}", "CAB-O-SIL TS-720 "par la société Cabot, "AEROSIL R972^{®}", "AEROSIL R974^{®}" par la société Degussa ; les argiles telles que la montmorillonite, les argiles modifiées telles que les bentones par exemple, l'hectorite stéaralkonium, la bentonite stéaralkonium , les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

La proportion totale en agent(s) épaississant(s) dans les compositions selon l'invention peut aller de à 0,01 à 15 % en poids, par rapport au poids total de la composition, de préférence de 0,05 à 10% et mieux de 0,1 à 5% en poids.

Selon un mode de réalisation particulier, la composition comprend un agent épaississant choisi parmi les bentones, en une teneur inférieure ou égale à 3% en poids par rapport au poids total de la composition.

### Matière colorante

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.
Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Les colorants hydrosolubles sont par exemple le jus de betterave, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.
Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon^{®}) (Orgasol^{®} de chez Atochem), de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} de la société Dow Corning) et les microbilles de résine de silicone (Tospearls^{®} de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

### Autres Additifs

La composition peut comprendre, en outre, d'autres ingrédients utilisés couramment dans les compositions cosmétiques. De tels ingrédients peuvent être choisis parmi les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Selon un autre aspect, l'invention a pour objet un produit de vernis à ongles comprenant : i) un récipient délimitant au moins un compartiment, ledit récipient étant fermé par un élément de fermeture et ii) une composition selon l'invention qui est disposée à l'intérieur dudit compartiment.

Le récipient peut être sous toute forme adéquate. Il peut être notamment sous forme d'une bouteille et peut être, au moins pour partie, en un matériau tel que le verre. Toutefois, des matériaux autres que le verre peuvent être utilisés comme les matériaux thermoplastiques tels que le PP ou le PE ou comme un métal.

L'élément de fermeture peut être couplé au compartiment par vissage en position fermée du récipient. Alternativement, le couplage entre l'élément de fermeture et le récipient peut se faire autrement que par vissage, notamment par encliquetage.

Le récipient est de préférence équipé d'un applicateur pouvant être sous forme d'un pinceau constitué d'au moins une touffe de poils. Alternativement, l'applicateur se présente sous forme autre qu'un pinceau, par exemple sous forme d'une spatule ou d'un embout en mousse.

Les exemples qui suivent illustrent de manière non limitative l'invention. Sauf indication contraire, les quantités indiquées sont des pourcentages massiques.

### Exemples

### Méthode de mesure de la viscosité

a/ La viscosité à 110°C du polymère est mesurée à l'aide d'un viscosimètre à cône plan de type BROOKFIELD CAP 1000+.
   Le cône-plan adapté est déterminé par l'homme du métier, sur la base de ses connaissances; notamment :
   - entre 50 et 500 mPa.s, on peut utiliser un cône 02
   - entre 500 et 1000 mPa.s : cône 03
   - entre 1000 et 4000 mPa.s : cône 05
   - entre 4000 et 10000 mPa.s : cône 06
b/ La viscosité à 25°C de la solution du polymère à 70% dans l'acétate de butyle est mesurée à l'aide d'un viscosimètre BROOKFIELD DV-I, à 30 tours/min en utilisant un mobile S62.

### Exemple 1 : Synthèse du pentaérythrityl benzoate/isophtalate/isostéarate

Dans un réacteur équipé d'une agitation mécanique, d'une arrivée d'argon et d'un système de distillation, on charge 227,5 g d'acide benzoïque, 72,8 g d'acide isostéarique et 118,3 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 18 heures. On refroidit à une température comprise entre 100 et 130°C puis on introduit 91 g d'acide isophtalique et on chauffe à nouveau progressivement jusqu'à 220°C pendant environ 11 heures.
On obtient ainsi 430 g de polycondensat pentaérythrityl benzoate/isophta-late/isostéarate sous la forme d'une huile épaisse qui se solidifie à température ambiante.

Le polycondensat présente les caractéristiques suivantes :
- Indice d'acide = 12,7
- Indice d'hydroxyle = 49
- η_{110°C} = 25,4 Poises (soit 2540 mPa.s)
- rapport entre le nombre de mole d'acide monocarboxylique aromatique et le nombre de mole d'acide monocarboxylique non aromatique : 7,28.

On prélève 420g de polycondensat obtenu ci-dessus, on le chauffe à 100-120°C et on coule lentement 180g d'acétate de butyle sous agitation puis on clarifie par filtration à chaud sur fritté n°2.
On obtient après refroidissement à température ambiante 600 g de solution de polycondensat à 70% dans l'acétate de butyle, se présentant sous la forme d'un liquide visqueux jaune pâle et possédant une viscosité à 25°C d'environ 800 centipoises (mPa.s)

### Exemple 2 : Vernis à ongles

| | |
|---|---|
| Solution à 70% en matières sèches du polymère de l'exemple 1 dans l'acétate de butyle | 17,8 |
| Nitrocellulose à 30 % d'alcool isopropylique (viscosité: E22 - 1/2 S, Idyl EMV de Bergerac) | 12,9 |
| Nitrocellulose à 30 % d'alcool isopropylique (Idyl E27 de Bergerac) | 5,54 |
| Copolymère acide adipique/anhydride triméllitique/ néopentyl glycol à 70% dans l'acétate de butyle (« Uniplex 670-P » de UNITEX CHEMICAL CORPORATION) | 2 |
| Acétyle citrate de tributyle | 0,8 |
| Résine epoxy toluènesulfonamide à 25% dans acétate de butyle | 2,42 |
| Hectorite modifiée stéaryl benzyl diméthyl ammonium | 2,08 |
| N-éthyl O,P-toluènesulfonamide | 1,61 |
| Condensat Oxyde d'éthylène/oxyde de propylène/ Oxyde d'éthylène (130E/300P/130E) commercialisé par UNIQEMA sous la référence SYNPERONIC PE/ L64 | 3 |
| Alcool n-butylique | 0,8 |
| Acétate de n-propyle | 12,1 |
| Alcool isopropylique | 0,81 |
| Acide citrique, 1 H2O | 0,09 |
| Acétate d'éthyle | 19,38 |
| Acétate de butyle | Qsp 100 |

| | |
|---|---|
| ⁽¹⁾ commercialisé par SHELL sous le nom de marque CARDURA 30^{®}. | |

Ce vernis après application sur les ongles, forme un film transparent très brillant.

### Exemples 3 et 4 : Vernis à ongles

On a préparé un vernis à ongles selon l'invention (exemple 3) et un vernis à ongles selon l'art antérieur (exemple 4) :

| | **Exemple 3** | **Exemple 4** |
|---|---|---|
| Solution à 70% en matières sèches du polymère de l'exemple 1 dans l'acétate de butyle | 17,6 | - |
| Nitrocellulose à 30 % d'alcool isopropylique (viscosité: E22 - 1/2 S, Idyl EMV de Bergerac) | 13,9 | 14,1 |
| Nitrocellulose à 30 % d'alcool isopropylique (viscosité: E24 - 1/8 S, Idyl EMV de Bergerac) | - | 1,99 |
| Nitrocellulose à 30 % d'alcool isopropylique (Azur E80 de Bergerac) | - | 1,18 |
| Nitrocellulose à 30 % d'alcool isopropylique (Idyl E27 de Bergerac) | 1,74 | - |
| Résine acétone formaldéhyde (SK de Degussa) | 0,175 | - |
| Copolymère acide adipique/anhydride triméllitique/ néopentyl glycol à 70% dans l'acétate de butyle (« Uniplex 670-P » de UNITEX CHEMICAL CORPORATION) | 1,97 | 2 |
| Acétyle citrate de tributyle | 3,67 | 0,87 |
| Résine epoxy toluènesulfonamide à 25% dans acétate de butyle | 2,61 | - |
| Résine alkyde glycérophtalique ésterifiée par acides gras ramifiés dans l'acétate d'éthyle à 70 % (CARDURA 30^{®} de SHELL) | - | 13,6 |
| Hectorite modifiée stéaryl benzyl diméthyl ammonium | 0,65 | 1,1 |
| N-éthyl O,P-toluènesulfonamide | 1,74 | 2,14 |
| Condensat Oxyde d'éthylène/oxyde de propylène/ Oxyde d'éthylène (13OE/30OP/13OE)commercialisé par UNIQEMA sous la référence SYNPERONIC PE/ L64 | 2,95 | - |
| Laque Red 7 | 0,2 | 0,2 |
| Oxyde de titane enrobé polyéthylène | 0,02 | 0,02 |
| Laque Red 34 | 0,55 | 0,55 |
| Polydiméthysiloxane (viscosité 350 cSt) | - | 0,199 |
| Alcol n-butylique | 0,87 | - |
| Acétate de n-propyle | 13 | - |
| Alcool isopropylique | 0,25 | 3,22 |
| Acide citrique, 1 H2O | 0,028 | 0,044 |
| Acétate d'éthyle | 20,9 | 27,18 |
| Acétate de butyle | Qsp 100 | Qsp 100 |

| | | |
|---|---|---|
| ⁽¹⁾ commercialisé par SHELL sous le nom de marque CARDURA 30^{®}. | | |

On a mesuré la brillance de chaque vernis selon le protocole suivant :
Sur une carte de contraste de marque LENETA et de référence FORM 1A PENOPAC, on étale une couche de 300 µm d'épaisseur humide de la composition dont on cherche à évaluer la brillance moyenne, à l'aide d'un étaleur automatique. La couche recouvre le fond blanc et la fond noir de la carte. On laisse sécher pendant 24 heures sur un banc thermostaté à 30°C puis on procède à la mesure de la brillance à 60° sur le fond blanc (3 mesures) et sur le fond noir (3 mesures) à l'aide d'un brillancemètre de marque BYK GARDNER et de référence microTRI-GLOSS.

On obtient les résultats suivants :

| | **Exemple 3** | **Exemple 4** |
|---|---|---|
| Brillance à 60° | 90,5 | 84,9 |

Le vernis à ongles selon l'invention présente une brillance bien supérieure à celle d'un vernis ne comprenant pas l'association d'un polycondensat de type alkyde modifié et d'un copolymère polyoxyalkylène.

## Revendications

1. Composition de vernis à ongles comprenant, dans un milieu solvant organique :
a) au moins un polycondensat susceptible d'être obtenu par réaction :
- de 15 à 30% en poids, par rapport au poids total du polycondensat, d'au moins un polyol comprenant 3 à 6 groupes hydroxyles;
- de 5 à 40% en poids, par rapport au poids total du polycondensat, d'au moins un acide monocarboxylique non aromatique, saturé ou insaturé, linéaire, ramifié et/ou cyclique, comprenant 6 à 32 atomes de carbone;
- de 10 à 55% en poids, par rapport au poids total du polycondensat, d'au moins un acide monocarboxylique aromatique comprenant 7 à 11 atomes de carbone, éventuellement en outre substitué par 1 à 3 radicaux alkyles, saturés ou insaturés, linéaires, ramifiés et/ou cycliques, qui comprennent 1 à 32 atomes de carbone;
- de 10 à 25% en poids, par rapport au poids total du polycondensat, d'au moins un acide polycarboxylique, saturé ou insaturé, voire aromatique, linéaire, ramifié et/ou cyclique, comprenant au moins 2 groupes carboxyliques COOH, notamment 2 à 4 groupes COOH; et/ou un anhydride cyclique d'un tel acide polycarboxylique, et
b) au moins un copolymère bloc polyoxyalkyléne choisi parmi :
- les copolymères bloc de formule (I) suivante :
HO-(CH2-CH2-O)n-(CxH2xO)m-(CH2-CH2-O)p-H,
dans laquelle n,m,p sont indépendamment des nombres entiers allant de 1 à 1000 et x est un nombre entier strictement supérieur à 2 et inférieur ou égal à 10,
- les copolymères comprenant au moins un bloc polyéthylène glycol et au moins un bloc polypropylène glycol, ledit copolymère présentant un poids moléculaire supérieur ou égal à 1000 g/mol
et leurs mélanges.

2. Composition selon la revendication 1, dans laquelle le polyol du polycondensat comprend 3 à 4 groupes hydroxyles.

3. Composition selon l'une des revendications précédentes, dans laquelle le polyol du polycondensat est un composé carboné, notamment hydrocarboné, linéaire, ramifié et/ou cyclique, saturé ou insaturé, comprenant 3 à 18 atomes de carbone, notamment 3 à 12, voire 4 à 10 atomes de carbone, et 3 à 6 groupes hydroxy (OH), et pouvant comprendre en outre un ou plusieurs atomes d'oxygène intercalés dans la chaîne (fonction éther).

4. Composition selon l'une des revendications précédentes, dans laquelle le polyol du polycondensat est un composé hydrocarboné saturé, linéaire ou ramifié, comprenant 3 à 18 atomes de carbone, notamment 3 à 12, voire 4 à 10 atomes de carbone, et 3 à 6 groupes hydroxy (OH).

5. Composition selon l'une des revendications précédentes, dans laquelle le polyol du polycondensat est choisi parmi, seul ou en mélange :
- les triols, tels que le 1,2,6-hexanetriol, le triméthyloléthane, le triméthylolpropane, le glycérol;
- les tétraols, tels que le pentaérythritol, l'érythritol, le diglycérol ou le ditriméthylolpropane;
- les pentols tels que le xylitol,
- les hexols tels que le sorbitol et le mannitol; ou encore le dipentaérythritol ou le triglycérol.

6. Composition selon l'une des revendications précédentes, dans laquelle le polyol du polycondensat est choisi parmi le glycérol, le pentaérythritol, le sorbitol et leurs mélanges; et encore mieux est du pentaérythritol.

7. Composition selon l'une des revendications précédentes, dans laquelle le polyol, ou le mélange de polyol, du polycondensat représente 16 à 28% en poids, et mieux 18 à 25% en poids, du poids total du polycondensat.

8. Composition selon l'une des revendications précédentes, dans laquelle l'acide monocarboxylique non aromatique du polycondensat est de formule RCOOH, dans laquelle R est un radical hydrocarboné saturé ou insaturé, linéaire, ramifié et/ou cyclique, comprenant 5 à 31 atomes de carbone, notamment 7 à 27 atomes de carbone, et encore mieux 9 à 19 atomes de carbone, voire 11 à 17 atomes de carbone.

9. Composition selon la revendication 8, dans laquelle le radical R est saturé.

10. Composition selon l'une des revendications 8 à 9, dans lesquelles le radical R est linéaire ou ramifié, et préférentiellement en C5-C31.

11. Composition selon l'une des revendications précédentes, dans laquelle l'acide monocarboxylique non aromatique du polycondensat est choisi parmi, seul ou en mélange:
- les acides monocarboxyliques saturés tels que l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
- les acides monocarboxyliques insaturés mais non aromatiques, tels que l'acide caproléique, l'acide undécylénique, l'acide dodécylénique, l'acide myristoléique, l'acide palmitoléique, l'acide oléique, l'acide élaidique, l'acide gondoïque, l'acide érucique.

12. Composition selon l'une des revendications précédentes, dans laquelle l'acide monocarboxylique non aromatique du polycondensat est choisi parmi l'acide 2-éthylhexanoïque, l'acide isooctanoïque, l'acide laurique, l'acide palmitique, l'acide isostéarique, et leurs mélanges, et encore mieux l'acide isostéarique seul.

13. Composition selon l'une des revendications précédentes, dans laquelle l'acide monocarboxylique non aromatique du polycondensat, ou le mélange desdits acides, représente 8 à 38% en poids, et mieux 10 à 35% en poids, du poids total du polycondensat.

14. Composition selon l'une des revendications précédentes, dans laquelle l'acide monocarboxylique aromatique du polycondensat est de formule R'COOH, dans laquelle R' est un radical hydrocarboné aromatique, comprenant 6 à 10 atomes de carbone, et en particulier les radicaux benzoïque et naphtoïque; ledit radical R' pouvant en outre être substitué par 1 à 3 radicaux alkyles, saturés ou insaturés, linéaires, ramifiés et/ou cycliques, comprenant 1 à 32 atomes de carbone, notamment 2 à 12, voire 3 à 8 atomes de carbone; et notamment choisis parmi méthyle, éthyle, propyle, isopropyle, butyle, isobutyle, terbutyle, pentyle, isopentyle, néopentyle, cyclopentyle, hexyle, cyclohexyle, heptyle, isoheptyle, octyle ou isooctyle.

15. Composition selon l'une des revendications précédentes, dans laquelle l'acide monocarboxylique aromatique du polycondensat est choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque.

16. Composition selon l'une des revendications précédentes, dans laquelle l'acide monocarboxylique aromatique du polycondensat est choisi parmi l'acide benzoïque, l'acide o-toluique, l'acide m-toluique, l'acide 1-naphtoïque, seuls ou en mélanges; et encore mieux l'acide benzoïque seul.

17. Composition selon l'une des revendications précédentes, dans laquelle l'acide monocarboxylique aromatique du polycondensat, ou le mélange desdits acides, représente 20 à 52% en poids, voire 22 à 52% en poids, et mieux 25 à 50% en poids, du poids total du polycondensat.

18. Composition selon l'une des revendications précédentes, dans laquelle l'acide polycarboxylique du polycondensat est choisi parmi les acides polycarboxyliques linéaires, ramifiés et/ou cycliques, saturés ou insaturés, voire aromatiques, comprenant 2 à 20 atomes de carbone, notamment 3 à 18, et encore mieux 4 à 12 atomes de carbone, voire 4 à 10 atomes de carbone; ledit acide comprenant au moins deux groupes carboxyliques COOH, de préférence de 2 à 4 groupes COOH.

19. Composition selon la revendication 18, dans laquelle ledit acide polycarboxylique du polycondensat est aliphatique saturé, linéaire et comprend 2 à 20 atomes de carbone, notamment 3 à 18 atomes de carbone, voire 4 à 12 atomes de carbone.

20. Composition selon la revendication 18, dans laquelle ledit acide polycarboxylique du polycondensat est aromatique et comprend 8 à 12 atomes de carbone.

21. Composition selon l'une des revendications 1 à 17, dans laquelle l'anhydride cyclique répond à l'une des formules suivantes : 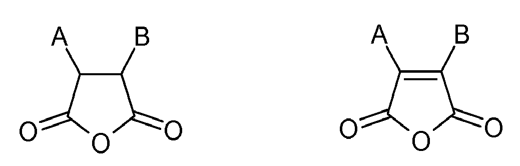 dans lesquelles les groupements A et B sont, indépendamment l'un de l'autre, :
- un atome d'hydrogène,
- un radical carboné, aliphatique, saturé ou insaturé, linéaire, ramifié et/ou cyclique, ou bien aromatique; comprenant 1 à 16 atomes de carbone, notamment 2 à 10 atomes de carbone, voire 4 à 8 atomes de carbone, notamment méthyle ou éthyle;
- ou bien A et B pris ensemble forment un cycle comprenant au total 5 à 7, notamment 6 atomes de carbone, saturé ou insaturé, voire aromatique.

22. Composition selon la revendication 21, dans laquelle A et B représentent un atome d'hydrogène ou forment ensemble un cycle aromatique comprenant au total 6 atomes de carbone.

23. Composition selon l'une des revendications précédentes, dans laquelle l'acide polycarboxylique du polycondensat ou son anhydride est choisi parmi, seul ou en mélange :
- les acides dicarboxyliques tels que l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque;
- les acides tricarboxyliques tels que l'acide cyclohexanetricarboxylique, l'acide trimellitique, l'acide 1,2,3-benzènetricarboxylique, l'acide 1,3,5-benzènetricarboxylique;
- les acides tétracarboxyliques tels que l'acide butanetétracarboxylique et l'acide pyroméllitique,
- les anhydrides cycliques de ces acides et notamment l'anhydride phtalique, l'anhydride trimellitique, l'anhydride maléïque et l'anhydride succinique.

24. Composition selon l'une des revendications précédentes, dans laquelle l'acide polycarboxylique du polycondensat ou son anhydride est choisi parmi l'anhydride phtalique et/ou l'acide isophtalique, et mieux l'acide isophtalique seul.

25. Composition selon l'une des revendications précédentes, dans laquelle l'acide polycarboxylique du polycondensat et/ou son anhydride cyclique représente 11 à 22% en poids, et mieux 12 à 20% en poids, du poids total du polycondensat.

26. Composition selon l'une des revendications précédentes, dans laquelle le polycondensat comprend en outre au moins une silicone à fonction hydroxyle (OH) et/ou carboxylique (COOH).

27. Composition selon la revendication 26, dans laquelle la silicone a une masse moléculaire moyenne en poids (Mw) comprise entre 300 et 20000, notamment 400 et 10 000, voire 800 et 4000.

28. Composition selon l'une des revendications 26 à 27, dans laquelle la silicone est de formule : 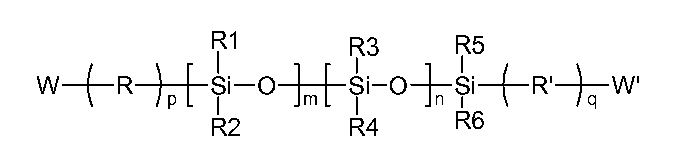 dans laquelle :
- W et W' sont, indépendamment l'un de l'autre, OH ou COOH; de préférence W=W';
- p et q sont, indépendamment l'un de l'autre, égaux à 0 ou 1,
- R et R' sont, indépendamment l'un de l'autre, un radical divalent carboné, notamment hydrocarboné, saturé ou insaturé, voire aromatique, linéaire, ramifié et/ou cyclique; comprenant 1 à 12 atomes de carbone, notamment 2 à 8 atomes de carbone, et comprenant éventuellement en outre 1 ou plusieurs hétéroatomes choisis parmi O, S et N, notamment O (éther);
notamment R et/ou R' peuvent être de formule -(CH₂)ₐ- avec a=1-12, et notamment méthylène, éthylène, propylène, phénylène;
ou bien de formule -[(CH₂)ₓ O]_{z}- avec x = 1, 2 ou 3 et z = 1-10; en particulier x=2 ou 3 et z=1-4; et mieux x=3 et z=1.
- R1 à R6 sont, indépendamment l'un de l'autre, un radical carboné linéaire, ramifié et/ou cyclique, saturé ou insaturé voire aromatique; comprenant 1 à 20 atomes de carbone, notamment 2 à 12 atomes de carbone; de préférence, R1 à R6 sont saturés ou bien aromatiques, et peuvent notamment être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
- m et n sont, indépendamment l'un de l'autre, des entiers compris entre 1 et 140, et sont tels que la masse moléculaire moyenne en poids (Mw) de la silicone est comprise entre 300 et 20 000, notamment entre 400 et 10 000, voire entre 800 et 4000.

29. Composition selon l'une des revendications 26 à 28, dans laquelle la silicone est choisie parmi, seule ou en mélange, les polyalkylsiloxanes α,ω-diol ou α,ω-dicarboxylique, et notamment les polydiméthysiloxanes α,ω-diol et les polydiméthylsiloxanes α,ω-dicarboxylique; les polyarylsiloxanes α,ω-diol ou α,ω-dicarboxylique et notamment les polyphénylsiloxanes α,ω-diol ou α,ω-dicarboxylique; les polyarylsiloxanes à fonctions silanol tels que le polyphénylsiloxane; les polyalkylsiloxanes à fonctions silanol tels que le polydiméthylsiloxane; les polyaryl/alkylsiloxanes à fonctions silanol tels que le polyphényl/méthylsiloxane ou encore le polyphényl/propylsiloxane.

30. Composition selon l'une des revendications 26 à 29, dans laquelle la silicone est choisie parmi les polydiméthysiloxanes α,ω-diol de masse moléculaire moyenne en poids (Mw) comprise entre 400 et 10 000, voire entre 500 et 5000, et notamment entre 800 et 4000.

31. Composition selon l'une des revendications 26 à 30, dans laquelle la silicone représente 0,1 à 15% en poids, notamment 1 à 10% en poids, voire 2 à 8% en poids, du poids total du polycondensat.

32. Composition selon l'une des revendications précédentes, dans laquelle le rapport entre le nombre de mole d'acide monocarboxylique aromatique et le nombre de mole d'acide monocarboxylique non aromatique est compris entre 1,2 et 8, en particulier entre 1,3 et 7,8, voire entre 1,4 et 7,5 et encore mieux entre 1,9 et 7.

33. Composition selon l'une des revendications précédentes, dans laquelle le polycondensat est susceptible d'être obtenu par réaction :
- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol; présent de préférence en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
présent de préférence en une quantité de 5 à 40% en poids, notamment 8 à 38% en poids, et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque;
présent de préférence en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul
ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque et l'anhydride succinique;
présent de préférence en une quantité de 10 à 25% en poids, notamment 11 à 22% en poids, et mieux 12 à 20% en poids, par rapport au poids total du polycondensat final.

34. Composition selon l'une des revendications précédentes, dans laquelle le polycondensat est susceptible d'être obtenu par réaction :
- d'au moins un polyol choisi parmi, seul ou en mélange, le glycérol, le pentaérythritol, le sorbitol et leurs mélanges, et encore mieux le pentaérythritol seul; présent en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide 2-éthylhexanoïque, l'acide isooctanoïque, l'acide laurique, l'acide palmitique, l'acide isostéarique, et leurs mélanges, et encore mieux l'acide isostéarique seul; présent en une quantité de 5 à 40% en poids, notamment 8 à 38% en poids, et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide 1-naphtoïque, et encore mieux l'acide benzoïque seul; présent en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul
ou en mélange, l'anhydride phtalique et l'acide isophtalique, et encore mieux l'acide isophtalique seul; présent en une quantité de 10 à 25% en poids, notamment 11 à 22% en poids, et mieux 12 à 20% en poids, par rapport au poids total du polycondensat final.

35. Composition selon l'une des revendications précédentes, dans laquelle le polycondensat présente au moins l'une des caractéristiques suivantes :
- un indice d'acide, exprimé en mg d'hydroxyde de potassium par g de polycondensat, supérieur ou égal à 8; notamment compris entre 8 et 40, et encore mieux compris entre 10 et 30; et/ou
- un indice d'hydroxyle exprimé en mg d'hydroxyde de potassium par g de polycondensat, supérieur ou égal à 30; notamment compris entre 30 et 100, et encore mieux compris entre 40 et 90.
- une viscosité, mesurée à 110°C, comprise entre 75 et 6000 mPa.s, notamment entre 80 et 5500 mPa.s, voire entre 90 et 5000 mPa.s, et encore mieux entre 200 et 4800 mPa.s;
- une solubilité dans l'acétate de butyle ou l'acétate d'éthyle, à raison d'au moins 50% en poids, à 25°C;
- une viscosité d'une solution du polymère dans l'acétate de butyle ou l'acétate d'éthyle, à 25°C, à une concentration de 70% en poids, comprise entre 100 et 1500 mPa.s, notamment entre 120 et 900 mPa.s.

36. Composition selon l'une des revendications précédentes, dans laquelle le polycondensat est présent en une teneur en matières sèches allant de 0,5 à 50% en poids, de préférence allant de 0,5 à 40% en poids, notamment de 1 à 25% en poids, et mieux de 5 à 20% en poids, par rapport au poids de la composition finale.

37. Composition selon la revendication 1, **caractérisé en ce que** le copolymère bloc polyoxyalkyléne répond à la formule suivante :
HO-(CH2-CH2-O)n-(CxH2xO)m-H
avec n et m des nombres entiers entre 1 et 1000 et x un nombre entier supérieur strictement à 2 et inférieur ou égal à 10.

38. Composition selon l'une des revendications précédentes, **caractérisée en ce que** le copolymère bloc polyoxyalkyléne est présent en une quantité allant de 0,1 à 15% en poids par rapport au poids total de la composition, de préférence de 0,5 à 10%, et mieux de 1 à 8% en poids.

39. Composition selon l'une des revendications précédentes, **caractérisée en ce que** le milieu solvant organique comprend au moins un solvant organique choisi parmi :
- les cétones liquides à température ambiante tels que les méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la □-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyl, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle;
- les carbonates tel que le carbonate de propylène, le carbonate de diméthyle,
- les acétales tels que le méthylal ;
- leurs mélanges.

40. Composition selon l'une des revendications précédentes, **caractérisée en ce que** le milieu solvant organique représente de 10 à 95% en poids, par rapport au poids total de la composition, de préférence de 15% à 80% en poids, et mieux de 60 à 80% en poids.

41. Composition selon l'une des revendications précédentes, **caractérisée en ce qu'**elle comprend au moins un agent épaississant.

42. Composition selon la revendication 41, **caractérisée en ce que** l'agent épaississant est choisi parmi les silices, notamment hydrophobes, les argiles telles que la montmorillonite, les argiles modifiées telles que les bentones, les alkyléther de polysaccharides et leurs mélanges.

43. Composition selon la revendication 41 ou 42, **caractérisée en ce que** l'agent épaississant est présent en une teneur allant de à 0,01 à 15 % en poids, par rapport au poids total de la composition, de préférence de 0,05 à 10% et mieux de 0,1 à 5% en poids.

44. Composition selon l'une des revendications précédentes, **caractérisée en ce qu'**elle comprend un polymère filmogène.

45. Composition selon la revendication précédente, **caractérisée en** en ce que le polymère filmogène est présent en une teneur en matières sèches allant de 0,1 % à 60 % en poids, par rapport au poids total de la composition, de préférence allant de 2 % à 50 % en poids, et mieux de 5 % à 30 % en poids.

46. Composition selon l'une des revendications précédentes, **caractérisée en ce qu'**elle comprend une matière colorante.

47. Composition selon la revendication précédente, **caractérisée en ce que** la matière colorante est présent en une teneur allant de 0,01 % à 50 % en poids, par rapport au poids de la composition, de préférence de 0,01 % à 30 % en poids.

48. Procédé cosmétique de maquillage ou de soin non thérapeutique des ongles comprenant l'application sur les ongles d'au moins une couche d'une composition de vernis à ongles selon l'une des revendications 1 à 47.

49. Utilisation d'une composition de vernis à ongles selon l'une des revendications 1 à 47, pour obtenir, après application sur les ongles, un film de vernis brillant.

50. Ensemble cosmétique comprenant : i) un récipient délimitant au moins un compartiment, ledit récipient étant fermé par un élément de fermeture et ii) une composition disposée à l'intérieur dudit compartiment, la composition étant conforme à l'une quelconque des revendications 1 à 47.
